# EUROPEAN PATENT APPLICATION

(11) **EP 4 730 346 A1**
(43) Date of publication of application: **22.04.2026**
(21) Application number: 25820385.0
(22) Date of filing: 28.05.2025
(51) Int. Cl.: G16C 20/70, G16C 20/80, G16C 20/40, G06N 3/0455, G06N 3/0895, G06N 3/0985, G06N 3/042

(54) **MOLECULAR STRUCTURE RECOGNITION SYSTEM, CONTROL METHOD THEREOF, AND METHOD OF TRAINING MOLECULAR STRUCTURE RECOGNITION SYSTEM**

(30) Priority: 04.06.2024 KR 20240073056; 26.02.2025 KR 20250025328
(71) Applicant: LG Management Development Institute Co., Ltd., Seoul 07336 (KR)
(72) Inventor: KIM, Ji Ye, Goyang-si Gyeonggi-do 10372 (KR); JO, Yeon Sik, Seoul 07791 (KR); JO, Ah Ra, Gimpo-si Gyeonggi-do 10113 (KR)
(74) Representative: BCKIP Part mbB
(86) International application number: PCT/KR2025/007257
(87) International publication number: WO 2025/254381

(57) **Abstract**

The present disclosure relates to a molecular structure recognition system, a control method thereof, and a learning method of a molecular structure recognition system. More specifically, the present disclosure relates to a learning method of a molecular structure recognition model of a molecular structure recognition system.

## Description

### [Technical Field]

The present disclosure relates to a molecular structure recognition system, a control method thereof, and a learning method of a molecular structure recognition system. More particularly, the present disclosure relates to a learning method of a molecular structure recognition model of a molecular structure recognition system.

### [Background Art]

Effectively searching and utilizing molecular structural formulas is a critical task in the fields of chemistry and pharmaceutical research. These molecular structural formulas are stored and present in an image form in various research documents, patents, academic papers, etc.

The molecular structural formulas in the image form are intuitive for human to understand, but pose significant limitations for automated machine searching and analysis. In particular, researchers developing new materials need to consult the conventional researches or conduct a search for prior researches and already invented molecular structural formulas to avoid infringing on the existing patents. However, when the molecular structural formulas are stored in the image form, it is impossible to search (or explore) the molecular structural formulas, resulting in significant difficulties in the research and analysis process.

To address this issue, artificial intelligence (Al) technologies (e.g., optical character recognition (OCR)-based AI models, AI algorithms, or the like) have recently been developed to enable the molecular structural formulas in the image form to be searchable by converting the molecular structure formulas into string formats according to sequence representation schemes. However, it is highly complicated to convert some molecular structural formulas into the string formats by the conventional string representation schemes. These techniques have limitations in converting complex molecular structures.

For example, the Markush structure serves as a scheme representing molecules that share the same backbone but allow diverse modifications, which may not be fully captured by the conventional linear string representation schemes.

As another example, since polymers include repetitive substructures, converting the polymers into a simple SMILES format may increase their complexity and lead to data loss.

Therefore, there is still a need for methods that enable artificial intelligence models to directly understand molecular structures based on images, just as humans understand molecular structural formulas as images.

### [Disclosure]

### [Technical Problem]

The present disclosure is directed to providing a molecular structure recognition system, a control method thereof, and a learning method of a molecular structure recognition system that may be usefully utilized in the fields of chemistry and pharmaceutical research.

More specifically, the present disclosure is directed to providing a molecular structure recognition model capable of enhancing the efficiency of molecular structure search and analysis in the fields of chemistry and pharmaceutical research.

In addition, the present disclosure is directed to providing a molecular structure recognition model capable of directly understanding and interpreting a molecular structure image.

Furthermore, the present disclosure is directed to providing a learning method for a molecular structure recognition model capable of training and recognizing a molecular structure from a molecular structure image itself, without converting the molecular structure image into a string.

### [Technical Solution]

According to an aspect of the present disclosure, a learning method of a molecular structure recognition system, performed cooperatively by a memory and at least one processor includes: inputting molecular structure images of each of a plurality of molecular structures to an image encoder; inputting strings according to a sequence representation scheme of each of the plurality of molecular structures to a molecule encoder; acquiring, from the image encoder, embedding vectors corresponding to the molecular structure images of each of the plurality of molecular structures; acquiring, from the molecule encoder, the embedding vectors corresponding to the strings according to the sequence representation scheme of each of the plurality of molecular structures; storing, in the memory, the embedding vectors corresponding to the molecular structure images of each of the plurality of molecular structures and the embedding vectors corresponding to the strings according to the sequence representation scheme of each of the plurality of molecular structures; and performing contrastive learning on a molecular structure recognition model of the molecular structure recognition system using the embedding vectors corresponding to the molecular structure images of each of the plurality of molecular structures and the embedding vectors corresponding to the strings according to the sequence representation scheme of each of the plurality of molecular structures that are stored in the memory.

The learning method may further include: constructing a training dataset including the molecular structure images of each of the plurality of molecular structures and the strings according to the sequence representation scheme of each of the plurality of molecular structures, in which the training dataset may be constructed such that the molecular structure images of each of the plurality of molecular structures and the strings according to the sequence representation scheme of each of the plurality of molecular structures form a pair.

At least one molecular structure image having respectively different visual appearances may be paired with each string according to the sequence representation scheme of each of the plurality of molecular structures.

The molecular structure images of each of the plurality of molecular structures may include the molecular structure images having the respectively different visual appearances generated using a predefined molecular structure image generation scheme, and in the constructing of the training dataset, the molecular structure images having the respectively different visual appearances while maintaining characteristics of each of the plurality of molecular structures may be generated.

The learning method may further include: specifying, from the training dataset, the molecular structure images of each of the plurality of molecular structures and the strings according to the sequence representation scheme of each of the plurality of molecular structures that form a pair, in which the plurality of molecular structures may include at least one of a first molecular structure and a second molecular structure.

The specifying may include: specifying a molecular structure image of the first molecular structure and a string according to a sequence representation scheme of the first molecular structure that form a pair; and specifying a molecular structure image of the second molecular structure and a string according to a sequence representation scheme of the second molecular structure that form a pair.

The inputting of the strings to the molecule encoder may include: converting atoms into nodes and converting bonds between the atoms into edges based on the strings according to the sequence representation scheme of each of the plurality of molecular structures to acquire a molecular graph of each of the plurality of molecular structures; and inputting the molecular graph of each of the plurality of molecular structures to the molecule encoder.

The acquiring of the molecular graph may include: converting atoms constituting a first molecular structure into nodes and converting bond relationships between the atoms constituting the first molecular structure into edges using a predefined tool to acquire a first molecular graph including the nodes and edges corresponding to the first molecular structure ; and converting atoms constituting a second molecular structure into nodes and converting bond relationships between the atoms constituting the second molecular structure into edges using the tool to acquire a second molecular graph including the nodes and edges corresponding to the second molecular structure, and in the inputting of the molecule encoder, the acquired first molecule graph and second molecule graph may be input to the molecule encoder.

In the molecule encoder, an embedding vector corresponding to the first molecule graph may be generated using the nodes and edges corresponding to the first molecule graph, in the molecule encoder, an embedding vector corresponding to the second molecule graph may be generated using the nodes and edges corresponding to the second molecule graph, and the embedding vector corresponding to the first molecule graph and the embedding vector corresponding to the second molecule graph generated from the molecule encoder may be acquired.

In the inputting of the molecular structure images to the image encoder, a molecular structure image of a first molecular structure and a molecular structure image of a second molecular structure may be input to the image encoder, and an embedding vector corresponding to the molecular structure image of the first molecular structure and an embedding vector corresponding to the molecular structure image of the second molecular structure may be acquired from the image encoder.

In the performing of the contrastive learning, the molecular structure recognition model may be trained using a preset loss function so as to maximize a similarity between a molecular structure image of a first molecular structure and a string according to a sequence representation scheme of the first molecular structure that form a pair, and the molecular structure recognition model may be trained using the loss function so as to maximize a similarity between a molecular structure image of a second molecular structure and the string according to the sequence representation scheme of the second molecular structure that form a pair.

According to another aspect of the present disclosure, a similarity between an embedding vector corresponding to the molecular structure image of the first molecular structure and an embedding vector corresponding to a first molecular graph may be maximized such that the embedding vector corresponding to the molecular structure image of the first molecular structure and the embedding vector corresponding to the first molecular graph may be included in a first embedding space, and a similarity between an embedding vector corresponding to the molecular structure image of the second molecular structure and an embedding vector corresponding to a second molecular graph may be maximized such that the embedding vector corresponding to the molecular structure image of the second molecular structure and the embedding vector corresponding to the second molecular graph may be included in a second embedding space.

In the performing of the contrastive learning, the molecular structure recognition model may be trained using a loss function so as to minimize a similarity between a molecular structure image of a first molecular structure and a string according to a sequence representation scheme of the first molecular structure that form a pair, and the molecular structure recognition model may be trained using the loss function so as to minimize a similarity between a molecular structure image of a second molecular structure and a string according to a sequence representation scheme of a second molecular structure that form a pair.

The similarity between the embedding vector corresponding to the molecular structure image of the second molecular structure and the embedding vector corresponding to the second molecular graphmolecular structure image of the second molecular structure, which are different from the similarity between the embedding vector corresponding to the molecular structure image of the first molecular structure and the embedding vector corresponding to the first molecular graph , may be minimized

The molecular structure recognition model may be trained to recognize the molecular structures corresponding to each of the plurality of molecular structures, recognize the molecular structures corresponding to the strings according to the sequence representation scheme of each of the plurality of molecular structures, or recognize molecular structures corresponding to a molecular graph of each of the plurality of molecular structures.

According to another aspect of the present disclosure, a molecular structure recognition system includes: a memory and at least one processor, in which the memory and the processor may cooperate to: input molecular structure images of each of a plurality of molecular structures to an image encoder, input strings according to a sequence representation scheme of each of the plurality of molecular structures to a molecule encoder; acquire, from the image encoder, embedding vectors corresponding to the molecular structure images of each of the plurality of molecular structures; acquire, from the molecule encoder, the embedding vectors corresponding to the strings according to the sequence representation scheme of each of the plurality of molecular structures; and perform contrastive learning on a molecular structure recognition model of the molecular structure recognition system using the embedding vectors corresponding to the molecular structure images of each of the plurality of molecular structures and the embedding vectors corresponding to the strings according to the sequence representation scheme of each of the plurality of molecular structures.

According to another aspect of the present disclosure, there is provided a program stored on a computer-readable recording medium, executed by one or more processes in an electronic device, in which the program may include instructions to perform: inputting molecular structure images of each of a plurality of molecular structures to an image encoder; inputting strings according to a sequence representation scheme of each of the plurality of molecular structures to a molecule encoder; acquiring, from the image encoder, embedding vectors corresponding to the molecular structure images of each of the plurality of molecular structures; acquiring, from the molecule encoder, the embedding vectors corresponding to the strings according to the sequence representation scheme of each of the plurality of molecular structures; storing, in the memory, the embedding vectors corresponding to the molecular structure images of each of the plurality of molecular structures and the embedding vectors corresponding to the strings according to the sequence representation scheme of each of the plurality of molecular structures; and performing contrastive learning on a molecular structure recognition model of the molecular structure recognition system using the embedding vectors corresponding to the molecular structure images of each of the plurality of molecular structures and the embedding vectors corresponding to the strings according to the sequence representation scheme of each of the plurality of molecular structures.

According to another aspect of the present disclosure, a control method of a molecular structure recognition system may include: receiving, from a user terminal, a user query related to a specific molecular structure among a plurality of molecular structures; processing the user query as input to a molecular structure recognition model trained based on contrastive learning between embedding vectors corresponding to molecular structure images of each of the plurality of molecular structures and embedding vectors corresponding to strings according to a sequence representation scheme of each of the plurality of molecular structures; extracting information on the specific molecular structure corresponding to the user query from the molecular structure recognition model; processing the extracted information on the specific molecular structure as input to a large language model (LLM); acquiring a response to the user query from the large language model; and providing the response to the user query to the user terminal.

According to another aspect of the present disclosure, a molecular structure recognition system may include: a control unit configured to receive, from a user terminal, a user query related to a specific molecular structure among a plurality of molecular structures, and process the user query as input to a molecular structure recognition model trained based on contrastive learning between embedding vectors corresponding to molecular structure images of each of the plurality of molecular structures and embedding vectors corresponding to strings according to a sequence representation scheme of each of the plurality of molecular structures, in which the control unit may extract information on the specific molecular structure corresponding to the user query using the molecular structure recognition model, process the extracted information on the specific molecular structure as input to a large language model (LLM), acquire a response to the user query from the large language model; and provide the response to the user query to the user terminal.

According to another aspect of the present disclosure, there is provided a program stored on a computer-readable recording medium, executed by one or more processors in an electronic device, in which the program may include instructions to perform: receiving, from a user terminal, a user query related to a specific molecular structure among a plurality of molecular structures; processing the user query as input to a molecular structure recognition model trained based on contrastive learning between embedding vectors corresponding to molecular structure images of each of the plurality of molecular structures and embedding vectors corresponding to strings according to a sequence representation scheme of each of the plurality of molecular structures; extracting information on the specific molecular structure corresponding to the user query from the molecular structure recognition model; processing the extracted information on the specific molecular structure as input to a large language model (LLM); acquiring a response to the user query from the large language model; and providing the response to the user query to the user terminal.

### [Advantageous Effect]

As described above, according to the molecular structure recognition system, the control method thereof, and the learning method of a molecular structure recognition system according to the present disclosure, it is possible to perform the contrastive learning on the molecular structure recognition model using the embedding vectors corresponding to the molecular structure image of each of the plurality of molecular structures and the embedding vectors corresponding to strings according to the sequence representation scheme of each of the plurality of molecular structures. The molecular structure recognition model trained through this may accurately recognize molecules even in various styles of molecular structure images and changes in orientation, and accurately distinguish subtle molecular structure differences even in molecular structure images including small differences such as atomic changes, bond changes, and chirality changes. In addition, the molecular structure recognition model may precisely analyze the similarities and differences between molecules by clearly identifying gradual differences (or degrees of modification) from small structural changes to large changes in molecules.

In addition, the molecular structure recognition system, the control method thereof method, and the learning method of a molecular structure recognition system according to the present disclosure may perform training to maximize the similarity between the pairs of the molecular structure images and the strings of the same molecular structure, and minimize the similarity between the pairs of the molecular structure images and the strings of respectively different molecular structures. The encoder of the molecular structure recognition model trained through this method may extract meaningful features including a deep understanding of the molecular structures from the molecular structural formula images without the need for direct image-to-molecule conversion. In addition, the encoder of the molecular structure recognition model can more accurately identify the details of the complex molecules and minimize the information loss during the translation process. In other words, according to the present disclosure, by enabling the direct understanding and interpretation of the molecular images, it is possible to significantly expand its applicability in the fields of chemistry and pharmaceutical research.

Furthermore, according to the molecular structure recognition system, the control method thereof, and the learning method of a molecular structure recognition system according to the present disclosure, it is possible to construct the training dataset by generating the molecular structure images having respectively different visual appearances while maintaining the characteristics of each of the plurality of molecular structures. In other words, according to the present disclosure, it is possible to provide the environment in which the model may perform training from various styles of molecular structure images while maintaining structural consistency of molecules. Through this, the molecular structure recognition model may utilize various styles of molecular structure images as training data, thereby maintaining consistent molecular recognition performance across diverse styles of molecular structure images without being biased toward a specific style of molecular structure image. In other words, the molecular structure recognition model may be widely utilized in patent search, new drug development, and chemical research, etc., by maintaining robust molecular recognition capabilities through training on various styles of molecular structure images.

In this way, the molecular structure recognition model according to the present disclosure may more clearly maintain structural similarities between molecules even in various styles of images. In particular, the encoder of the molecular structure recognition model may berobust to style changes and effectively distinguish the molecular structures. In addition, it is possible to achieve the strong generalization performance even on untrained new molecular structures and effectively recognize and search molecules through the image embedding. In other words, the molecular structure recognition model according to the present disclosure may easily understand the structural characteristics of molecules from the molecular structure image itself, just as humans easily understand the molecular structure images, thereby effectively extracting molecular-related features even for complex molecular structures.

### [Description of Drawings]

FIG. 1 is a conceptual diagram for describing a molecular structure recognition system according to the present disclosure.
FIGS. 2A and 2B are conceptual diagrams for describing a training dataset according to the present disclosure.
FIGS. 3A and 3B are conceptual diagrams for describing a molecular structure recognition model of a molecular structure recognition system according to the present disclosure.
FIG. 4 is a flowchart for describing a learning method of a molecular structure recognition system according to the present disclosure.
FIGS. 5A, 5B, 6A, 6B, and 7 are conceptual diagrams for describing the learning method of a molecular structure recognition system according to the present disclosure.
FIG. 8 is a flowchart for describing a control method of a molecular structure recognition system according to the present disclosure.
FIGS. 9 and 10 are conceptual diagrams for describing the control method of a molecular structure recognition system according to the present disclosure.

### [Mode for Disclosure]

Hereafter, embodiments disclosed in the present specification will be described in detail with reference to the accompanying drawings and the same or similar components are given the same reference numerals regardless of the numbers of figures and are not repeatedly described. In addition, terms "module" and "unit" for components used in the following description are used only to easily make the disclosure. Therefore, these terms do not have meanings or roles that distinguish from each other in themselves. Further, when it is determined that a detailed description for the related known art in describing embodiments disclosed in the present specification may obscure the gist of the present disclosure, a detailed description thereof will be omitted. Further, it should be understood that the accompanying drawings are provided only in order to allow embodiments disclosed in the present specification to be easily understood, and the spirit of the present disclosure is not limited by the accompanying drawings, but includes all the modifications, equivalents, and substitutions included in the spirit and the scope of the present disclosure.

Terms including ordinal numbers such as "first", "second", etc., may be used to describe various components, but the components are not to be construed as being limited to the terms. The terms are used to distinguish one component from another component.

It is to be understood that when one element is referred to as being "connected to" or "coupled to" another element, it may be connected directly to or coupled directly to another element or be connected to or coupled to another element, having the other element intervening therebetween. On the other hand, it should be understood that when one element is referred to as being "connected directly to" or "coupled directly to" another element, it may be connected to or coupled to another element without the other element interposed therebetween.

Singular expressions are intended to include plural expressions unless the context clearly indicates otherwise.

It will be further understood that terms "include", "have", or the like used in the present specification specify the presence of features, numerals, steps, operations, components, parts mentioned in the present specification, or combinations thereof, but do not preclude the presence or addition of one or more other features, numerals, steps, operations, components, parts, or combinations thereof.

The present disclosure relates to a molecular structure recognition system, a control method thereof, and a learning method of a molecular structure recognition system. The molecular structure recognition system, the control method thereof, and the learning method of a molecular structure recognition system according to the present disclosure may be usefully utilized in various situations. For example, the present disclosure may be usefully utilized in the field of chemical research for designing new materials or developing new drugs. The present disclosure relates to recognizing a molecular structure corresponding to a molecular structure image of a molecular structure, recognizing a molecular structure corresponding to a string according to a sequence representation scheme of the molecular structure, or recognizing a molecular structure corresponding to a molecular graph of the molecular structure, and provides a molecular structure recognition system, a control method, and a learning method. The molecular structure recognition system, the control method thereof, and the learning method of a molecular structure recognition system according to the present disclosure are implemented based on a "molecular structure recognition model". Hereinafter, for convenience of description, the system, the method, and the learning method will not be separately named, but will be unified and referred to as the "molecular structure recognition model".

Meanwhile, the molecular structure recognition model according to the present disclosure may be applied to various industries and services. For example, the molecular structure recognition model may be usefully applied to systems based on at least one of a language model (LM) and a large language model (LLM). Recently, with the advancement of deep learning technology, generative AI technology has been attracting attention. More specifically, generative AI models may generate new data in various forms, such as text, images, speech, and the like, from given data, and provide a new level of application potential beyond simply classifying or predicting existing data. The molecular structure recognition model according to the present disclosure may also be applied to systems implemented based on such generative AI models, and usefully utilized in various fields, including designing new materials, developing pharmaceuticals, and conducting molecular structure search and analysis.

The molecular structure recognition system according to the present disclosure includes the molecular structure recognition model capable of directly understanding and interpreting molecular structure images. The present disclosure aims to provide the molecular structure recognition model that may enhance the efficiency of the molecular structure search and analysis in the fields of chemistry and pharmaceutical research. In particular, the molecular structure recognition model according to the present disclosure may train and recognize molecular structures from a molecular structure image itself, without converting the molecular structure image into a string.

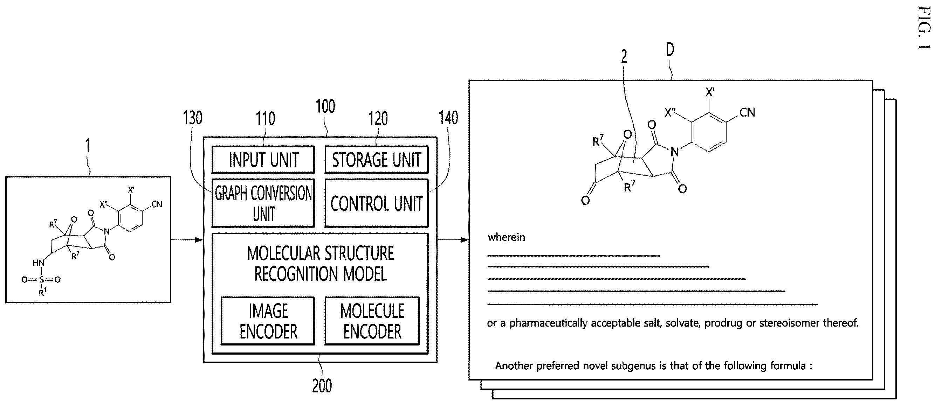

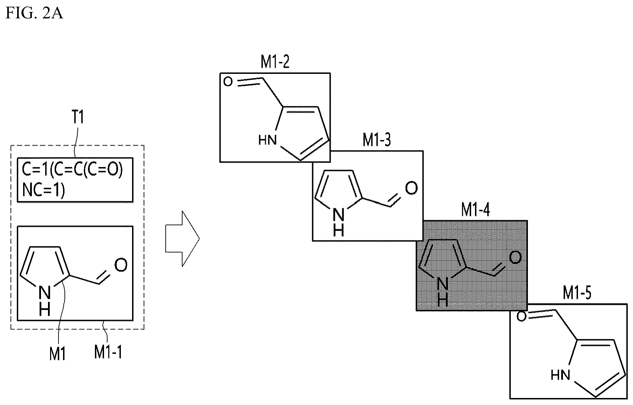

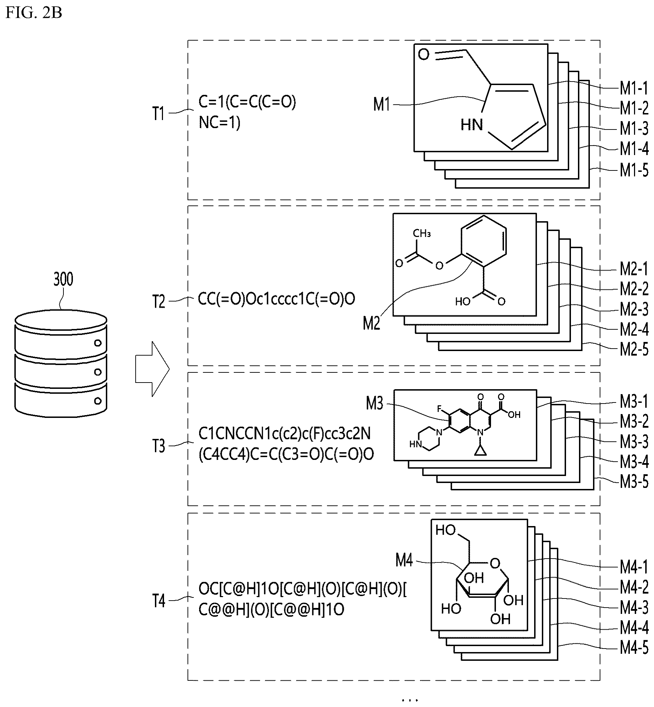

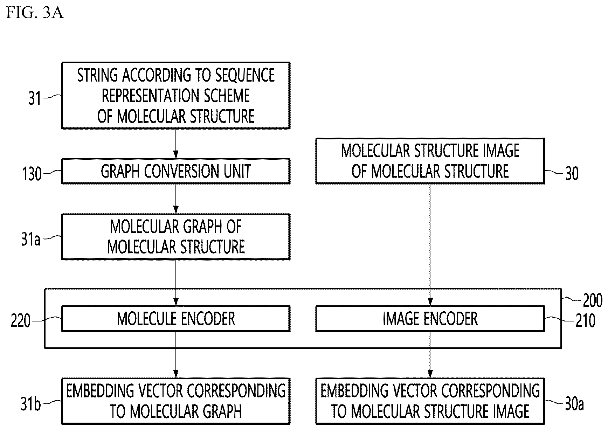

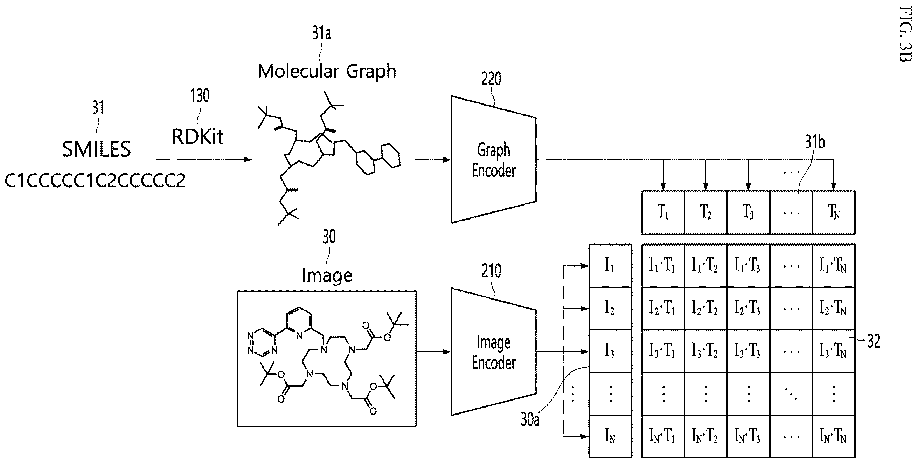

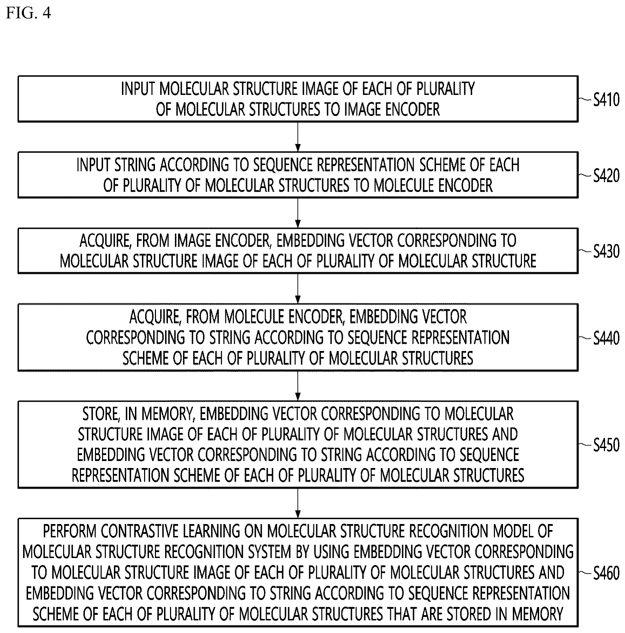

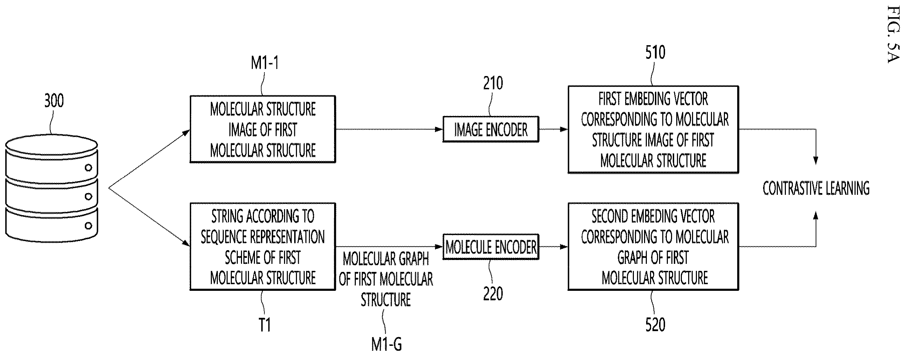

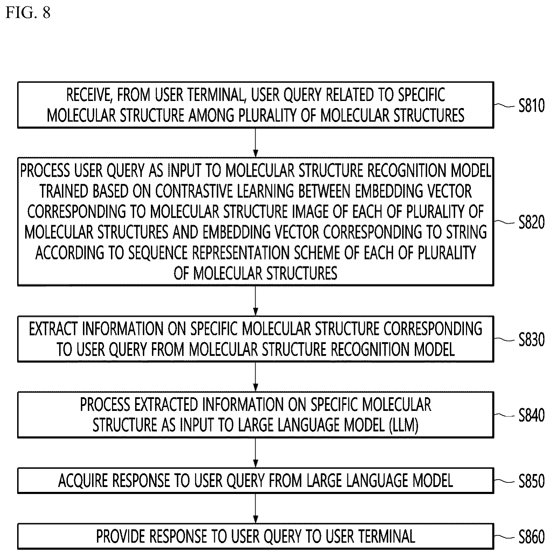

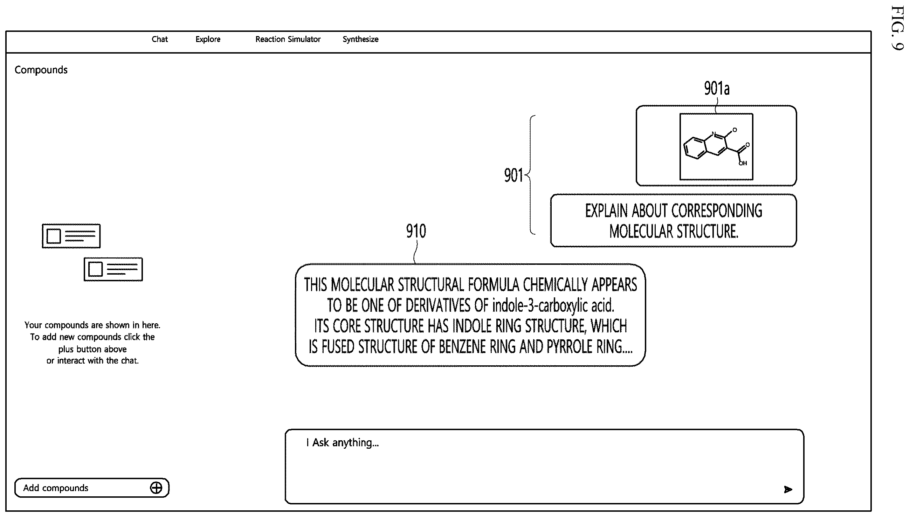

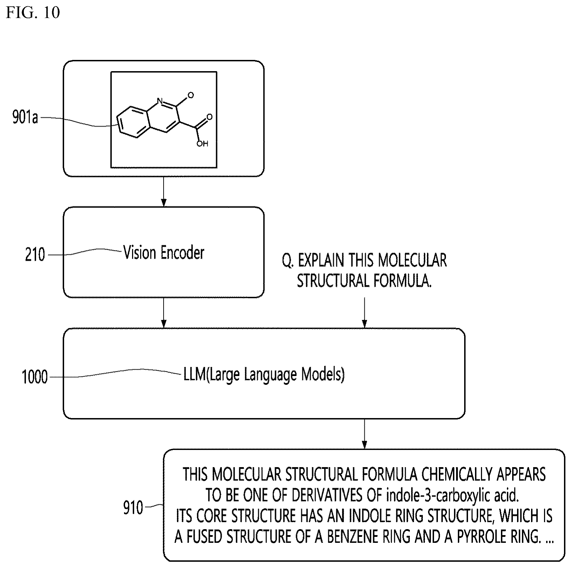

Hereinafter, the present disclosure will be examined in more detail with the accompanying drawings. FIG. 1 is a conceptual diagram for describing a molecular structure recognition system according to the present disclosure. FIGS. 2A and 2B are conceptual diagrams for describing a training dataset according to the present disclosure, and FIGS. 3A and 3B are conceptual diagrams for describing a molecular structure recognition model of the molecular structure recognition system according to the present disclosure. FIG. 4 is a flowchart for describing a learning method of a molecular structure recognition system according to the present disclosure, and FIGS. 5A, 5B, 6A, 6B, and 7 are conceptual diagrams for describing the learning method of a molecular structure recognition system according to the present disclosure. Furthermore, FIG. 8 is a flowchart for describing a control method of a molecular structure recognition system according to the present disclosure, and FIGS. 9 and 10 are conceptual diagrams for describing the control method of a molecular structure recognition system according to the present disclosure.

Meanwhile, as described above, when the molecular structure recognition system 100 according to the present disclosure is a system that recognizes a molecular structure corresponding to a molecular structure image of a molecular structure, recognizes a molecular structure corresponding to a string according to a sequence representation scheme of the molecular structure, or recognizes a molecular structure corresponding to a molecular graph of the molecular structure, some of the components described in FIG. 1 can be excluded.

First, as illustrated in FIG. 1, a molecular structure recognition system 100 according to the present disclosure may include at least one of an input unit 110, a storage unit 120, a graph conversion unit 130, a control unit 140, and a molecular structure recognition model 200.

The molecular structure recognition system 100 according to the present disclosure may include at least one processor and at least one memory including computer program code. In this case, the memory may function as the storage unit 120. In the present disclosure, the memory and the program code may cooperate with the processor to perform a series of processes described below.

Although not illustrated, the molecular structure recognition system 100 according to the present disclosure may include one or more processors, in which the processors may include one or more general-purpose processors and/or one or more special-purpose processors (e.g., a digital signal processor, a tensor processing unit (TPU), a graphics processing unit (GPU), a neural network processing unit (NPU), an application-specific integrated circuit (ASIC), etc.). One or more processors may be configured to execute instructions stored (or included) in the storage unit 120, computer-readable instructions, and/or other instructions described herein. The molecular structure recognition method and system according to the present disclosure may perform data processing to be described below through cooperation between a memory and at least one processor. The processor may perform a series of operations and data processing using data and information stored in the memory. In this case, the memory may be a component of the storage unit 120.

Meanwhile, the input unit 110 serves as a means for data input, and may be implemented in various forms. For example, the input unit 110 may be configured to receive the user input. The input unit 110 may be configured to receive user input (or user query) from a user terminal. Here, "receiving the input" may mean receiving an input signal (or selection signal) corresponding to the user input based on the fact that the input is made by the user through the configuration of the input unit provided in the user terminal.

In addition, in the present disclosure, the input unit 110 does not necessarily refer to a hardware means, but may be understood as a channel for receiving input from a user.

The input unit 110 may also be referred to as a user interface module. The input unit 110 may include a touch screen, a computer mouse, a keyboard, a keypad, a touch pad, a trackball, a joystick, a voice recognition module, or other similar devices. However, in the present disclosure, the type of the input unit 110 is not limited.

Here, the user input may include documents, text, images (or videos), speech, etc. In this case, the molecular structure recognition system 100 may further include a module that converts speech into text.

Next, the storage unit (120, or memory) serves to store various data related to the present disclosure and may include one or more non-transitory computer-readable storage media that may be read and/or accessed by at least one of the one or more processors.

One or more computer-readable storage media may include volatile and/or nonvolatile storage components, such as optical, magnetic, organic, or other memory or disk storage devices. In some examples, the storage unit 120 may be implemented using a single physical device (e.g., a single optical, magnetic, organic, or other memory, or disk storage device), while in other examples, the storage unit 120 may be implemented using two or more physical devices.

The storage unit 120 may include computer-readable instructions and additional data. The storage unit 120 may include storage necessary to perform at least some of the methods, scenarios, and techniques described herein and/or at least some of the functions of the devices and networks.

Furthermore, at least a portion of the storage unit 120 may be cloud storage or a cloud server. The storage unit 120 may store data corresponding to the user input received from the input unit 110 and at least a portion of a training data.

That is, the storage unit 120 may be sufficient as long as it has a space storing information necessary for the operation of the molecular structure recognition system 100 according to the present disclosure, and it may be understood that there are no physical space restrictions.

Furthermore, the storage unit 120 may store a computer program including computer program instructions. Furthermore, the storage unit 120 may store a computer program including computer program instructions that control the operation of the system 100 or the operation of the control unit 140 when the computer program instructions are loaded onto the processor of the system 100.

Next, the graph conversion unit 130 may be configured to generate a molecular graph of each of the plurality of molecular structures by converting atoms into nodes and converting bonds between the atoms into edges based on strings (e.g., SMILES, InChI, etc.) according to a sequence representation scheme of each of the molecular structures.

More specifically, the graph conversion unit 130 may convert the atoms constituting the molecular structure into the nodes and bond relationships between the atoms constituting the molecular structure into the edges, thereby generating the molecular graph including the nodes and edges corresponding to the molecular structure.

This graph conversion unit 130 may include at least one tool used in cheminformatics to handle and analyze the molecular structures. For example, the graph conversion unit 130 may include at least one of RDKit and Indigo, which are used for molecular structure (or chemical structure) analysis, molecular structure representation, molecular structure visualization, substructure search, molecular fingerprint generation, physicochemical property prediction, chemical reaction processing, etc. In addition, in the present disclosure, the graph conversion unit 130 may also be referred to as a "predefined tool", "RDKit", "Indigo", or the like.

Next, the control unit 140 may control the overall operation of the molecular structure recognition system 100 related to the present disclosure. The control unit 140 may process signals, data, information, etc., input or output through the components of the molecular structure recognition system 100 described above, or perform a series of data processing to provide or process appropriate information and functions to a user. The control unit 140 may be physically implemented by the processor described above.

Meanwhile, the control unit 140 may construct a training dataset to be used in the learning process of the molecular structure recognition model 200.

In the present disclosure, the diversity of the training data may be increased by generating various styles of molecular structure images for the same molecule. That is, in the present disclosure, to enable the molecular structure recognition model 200 to train an essential structure of a molecule without relying on a specific style or visual representation, various styles of molecular structure images are generated for the same molecule, enabling the molecular structure recognition model 200 to train consistent representations for various styles of molecular structure images.

In the present disclosure, this scheme (or technique, method, etc.) may also be referred to as "diverse positive molecular image views (DPV)". The DPV may be understood as a scheme that generates various styles of images for the same molecule to provide various positive samples for the same molecular structure when training matching between the molecular structure image and the molecular graph (or the string of the molecular structure). In the present specification, for convenience of description, the DPV is referred to as a "predefined molecular structure image generation scheme".

The predefined molecular structure image generation scheme (or technique, method, etc.) may include at least one of various rendering techniques, tools, image generation algorithms, or image transformation or augmentation techniques to ensure various visual appearances (or various styles) of molecular structure images.

In this regard, molecules (or molecular structures) may have unique characteristics, such as bonding schemes, atomic arrangements, physical and chemical properties, molecular structures, or the like. In addition, even for identical molecules, rendering styles may vary (e.g., presence or absence of atomic number display, line thickness, color, etc.). In this case, modifications such as cropping, rotating, or flipping a molecular structure image may misrepresent molecules or modify structural characteristics (or chemical and physical properties, or unique features) of the molecules.

Therefore, the predefined molecular structure image generation scheme of the present disclosure may include techniques such as color variation (or modification), line thickness and size adjustment, contrast adjustment (or brightness adjustment), Gaussian blur, and noise addition, or various chemical structure rendering tools (or software), which enable the generation of molecular structure images having respectively different visual appearances while maintaining the characteristics of the molecular structure.

Such a predefined molecular structure image generation scheme may include, for example, at least one of the predefined tool (e.g., "RDKit: a tool that represents atoms and bonds as lines and applies black and white or simple colors", "Indigo: a tool that applies line thickness, colors, and styles different from those of RDKit", etc.) and a predefined augmentation technique (e.g., soft augmentation (SoftAug), hard augmentation (HardAug), etc.).

The control unit 140 may construct a training dataset including molecular structure images of each of the plurality of molecular structures and strings according to a sequence representation scheme of each of the plurality of molecular structures.

In this case, the control unit 140 may use (or utilize, use, etc.) at least one of the strings according to the sequence representation scheme of the molecular structure and/or the molecular structure images of the molecular structures as data for generating molecular structure images having respectively different visual appearances. In this case, the molecular structure images used in the process of generating the molecular structure images having respectively different visual appearances may be images collected from various sources (e.g., databases, web crawling, APIs, servers linked to the molecular structure recognition system 100, external servers, etc.) or at least one image generated using the predefined tool.

Specifically, the control unit 140 may generate at least one molecular structure image having respectively different visual appearances for each of the plurality of molecular structures using the predefined molecular structure image generation scheme.

As described above, the control unit 140 may generate the molecular structure images having respectively different visual appearances for each of the plurality of molecular structures while maintaining the characteristics (e.g., structural characteristics or chemical characteristics, etc.) of each of the plurality of molecular structures.

In an embodiment, as illustrated in FIG. 2A, the control unit 140 may generate a plurality of molecular structure images M1-2, M1-3, M1-4, and M1-5 having respectively different visual appearances for a specific molecular structure M1 while maintaining the characteristics of a specific molecular structure (or the first molecular structure M1) among the plurality of molecular structures using the predefined molecular structure image generation scheme.

In this case, at least some M1-2 and M1-5 of the plurality of molecular structure images M1-2, M1-3, M1-4, and M1-5 having respectively different visual appearances generated for the first molecular structure M1 may be images generated by inputting a string T1 according to the sequence representation scheme of the first molecular structure M1 to the predefined tool (e.g., RDKit, Indigo, etc.). That is, some of the images M1-2 and M1-5 may be images generated using the predefined tool for the string T1 of the first molecular structure M1.

In addition, at least some M1-2 and M1-5 of the plurality of molecular structure images M1-2, M1-3, M1-4, and M1-5 having respectively different visual appearances generated for the first molecular structure M1 may be images generated by applying a predefined augmentation technique (e.g., soft augmentation (SoftAug), hard augmentation (HardAug), etc.) to the molecular structure image M1-1 of the first molecular structure. In this case, the molecular structure image M1-1 of the first molecular structure to which the augmentation technique is applied may be an image collected from various sources or an image generated using the predefined tool.

In another embodiment, the control unit 140 may generate the plurality of molecular structure images having respectively different visual appearances for the second molecular structure M2 while maintaining the characteristics of the second molecular structure M2 among the plurality of molecular structures using the predefined molecular structure image generation scheme.

In this case, at least some of the plurality of molecular structure images having respectively different visual appearances generated for the second molecular structure M2 may be images generated by inputting a string T2 according to the sequence representation scheme of the second molecular structure to the predefined tool. That is, some of the images may be images generated using the predefined tool for the string T2 of the second molecular structure.

In addition, at least some of the images of the plurality of molecular structures having respectively different visual appearances generated for the second molecular structure M2 may be images generated by applying a predefined augmentation technique to the molecular structure image of the second molecular structure. In this case, the molecular structure image of the second molecular structure to which the augmentation technique is applied may be an image collected from various sources or an image generated using the predefined tool.

Furthermore, according to the embodiment described above, the training dataset including the molecular structure images of each of the plurality of molecular structures and the strings according to the sequence representation scheme of each of the plurality of molecular structures may be constructed.

The control unit 140 may construct the training dataset by pairing the molecular structure images of each of the plurality of molecular structures and the strings according to the sequence representation scheme of each of the plurality of molecular structures. For example, as illustrated in FIG. 2B, a training dataset 300 may be constructed such that the molecular structure images of each of the plurality of molecular structures M1, M2, M3, and M4 and the strings T1, T2, T3, and T4 according to the sequence representation scheme of each of the plurality of molecular structures form a pair (or pairs).

In this case, each string T1, T2, T3, and T4 according to the sequence representation scheme of each of the plurality of molecular structures included in the training dataset 300 may be paired with at least one molecular structure image having respectively different visual appearances.

The molecular structure images having respectively different visual appearances may include at least one of a molecular structure image corresponding to data used to generate the molecular structure images having respectively different visual appearances and a molecular structure image generated using the predefined molecular structure image generation scheme.

For example, a plurality of images M1-1, M1-2, M1-3, M1-4, and M1-5 having respectively different visual appearances in the string T1 according to a sequence representation scheme of a first molecular structure may form a pair, and a plurality of images M2-1, M2-2, M2-3, M2-4, and M2-5 having respectively different visual appearances in the string T2 according to a sequence representation scheme of a second molecular structure may form a pair.

For another example, a plurality of images M3-1, M3-2, M3-3, M3-4, and M3-5 having respectively different visual appearances in a string T3 according to a sequence representation scheme of a third molecular structure may form a pair, and a plurality of images M4-1, M4-2, M4-3, M4-4, and M4-5 having respectively different visual appearances in a string T4 according to a sequence representation scheme of a fourth molecular structure may form a pair.

Accordingly, the molecular structure images of each of the plurality of molecular structures paired with strings T1, T2, T3, and T4 of each of the plurality of molecular structures may include the molecular structure images having respectivelydifferent visual appearances generated using the predefined molecular structure image generation scheme. The molecular structure images having respectively different visual appearances may include various images (synthetic images) generated while maintaining the characteristics of each molecular structure.

Meanwhile, the molecular structure recognition model 200 of the present disclosure may be trained to recognize a molecular structure corresponding to the molecular structure image of the molecular structure, a molecular structure corresponding to the string according to the sequence representation scheme of the molecular structure, or a molecular structure corresponding to the molecular graph of the molecular structure.

The molecular structure recognition model 200 may include an image encoder 210 and a molecule encoder 220 (or graph encoder). In the present disclosure, the molecular structure recognition model 200 may also be referred to as a "molecular understanding model", "molecular structure understanding model", "molecular recognition model", "molecular analysis model", "molecular structure analysis model", etc.

As illustrated in FIGS. 3A and 3B, when a molecular structure image 30 of a molecular structure is input, the image encoder 210 may be configured to generate (or output) an embedding vector 30a (or feature) corresponding to the molecular structure image 30. For example, the image encoder 210 may convert (or divide) the molecular structure image 30 into units of patches and then process (or embed) each patch to extract features (i.e., embedding vectors) of the molecular structure.

The embedding vector 30a corresponding to the molecular structure image 30 includes vector values that include important features (or characteristics) of the molecular structure image, and may be represented as a vector such as 512 dimensions, 1024 dimensions, or the like. For example, as illustrated in FIG. 3B, in the embedding vector 30a corresponding to the molecular structure image 30, "I1, I2, I3", etc., are vectors for each molecule image, and may be vector expressions extracted from the molecular structure image. Here, I1 may refer to an image vector of a first molecule, and I2 may refer to an image vector of a second molecule.

The image encoder 210 may be based on various artificial intelligence models (or networks). For example, the image encoder 210 may be a vision transformer (ViT)-based encoder.

When a molecular graph (e.g., 31a) for a molecular structure is input, the molecule encoder 220 may be configured to generate an embedding vector 31b (or feature) corresponding to the molecular graph 31a for the molecular structure. In this case, the graph conversion unit 130 may convert a string 31 (e.g., "C1CCCCC1C2CCCCC2") according to the sequence representation scheme of the molecular structure into the molecular graph 31a expressed by nodes (atoms) and edges (bonds), and input the converted molecular graph 31a to the molecule encoder 220. For example, the molecule encoder 220 may train structural information (e.g., node and edge information) of the molecular structure included in the molecular graph 31a using a message passing technique, and generate the embedding vector 31b that takes into account the surrounding environment (connectivity) of each atom constituting the molecular structure.

The embedding vector 31b corresponding to the molecular graph 31a may include vector values that include important features of the molecular graph. For example, as illustrated in FIG. 3B, in the embedding vector 31b, corresponding to the molecular graph 31a, "T1, T2, TN", and the like may be unique vectors reflecting the characteristics of each molecule and may be vector representations extracted from the molecular graph. Here, T1 may refer to the vector representation of the first molecule, and T2 may refer to the vector representation of the second molecule.

This molecule encoder 220 may be based on various artificial intelligence models (or networks). For example, the molecule encoder 220 may be a graph isomorphism network (GIN)-based encoder.

Furthermore, in the present disclosure, the contrastive learning may be performed on the molecular structure recognition model 200 such that, when the graph representation T and the molecular structure image representation I correspond to the same molecule, they are located in a close vector space, and when the graph representation T and the molecular structure image representation I correspond to different molecules, they are located at a distant position. For example, the control unit 140 may generate a similarity matrix 32 by calculating an inner product of each image vector I_i and each molecular graph vector T_j. Each element (e.g., I_n and T_j) of the similarity matrix 32 may represent a similarity between an ith image and a jth molecule. In this case, when the molecular structure image and the molecular graph correspond to the same molecule, the values of each element I_i and T_j may be extracted to be large, and when the molecular structure image and the molecular graph correspond to different molecular structures, the values of each element may be trained to be small. In other words, the molecular structure recognition model 200 may be trained through the contrastive learning to be close to a positive pair corresponding to a molecular structure image and a molecular graph of a specific molecular structure to be trained, and far from a negative pair corresponding to a molecular structure image and a molecular graph of a molecular structure different from the specific molecular structure.

Meanwhile, the present disclosure is directed to providing the molecular structure recognition system, the control method thereof, and the learning method of a molecular structure recognition system. More specifically, the present disclosure is directed to provide the molecular structure recognition model that may increase the efficiency of the molecular structure search and analysis in the fields of chemical and pharmaceutical research. Hereinafter, the learning method of a molecular structure recognition model 200 will be described in more detail.

As described above, the training dataset 300 according to the present disclosure may be constructed such that the molecular structure images of each of the plurality of molecular structures and the strings according to the sequence representation scheme of each of the plurality of molecular structures form a pair. In this case, the molecular structure images of each of the plurality of molecular structures included in the training dataset 300 may be understood to include at least one molecular structure image having respectively different visual appearances.

The control unit 140 may specify, from the training dataset 300, the molecular structure image of the first molecular structure and the string according to the sequence representation scheme of the first molecular structure that form a pair, and the molecular structure image of the second molecular structure and the string according to the sequence representation scheme of the second molecular structure that form a pair.

In this case, each of the molecular structure image of the first molecular structure and the molecular structure image of the second molecular structure may include the plurality of the molecular structure images having respectively different visual appearances.

Accordingly, in the present disclosure, the molecular structure image input to the molecular structure recognition model 200 may be understood as at least one of the molecular structure images having respectively different visual appearances. That is, any one of the molecular structure images having respectively different visual appearances and the string may be input to the molecular structure recognition model 200 so that the molecular structure recognition model 200 may perform consistent learning on the molecular structure images having respectively different visual appearances generated for the same molecular structure.

First, the control unit 140 may input, to the molecular structure recognition model 200, the molecular structure images of each of the plurality of molecular structures and the strings of each of the plurality of molecular structures that form a pair. More specifically, the control unit 140 may input the molecular structure images of each of the plurality of molecular structures to the image encoder 210.

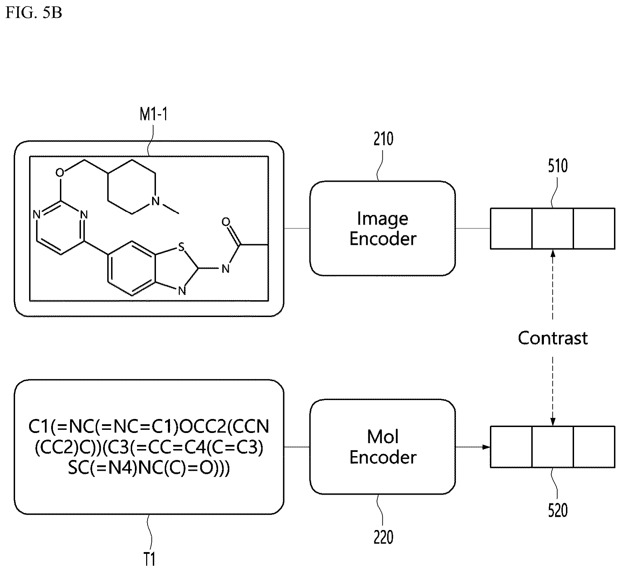

As illustrated in FIGS. 5A and 5B, the control unit 140 may input the molecular structure image M1-1 of the specified first molecular structure to the image encoder 210. For example, the control unit 140 may input at least one M1-1 of the plurality of molecular structure images having respectively different visual appearances of the first molecular structure to the image encoder 210.

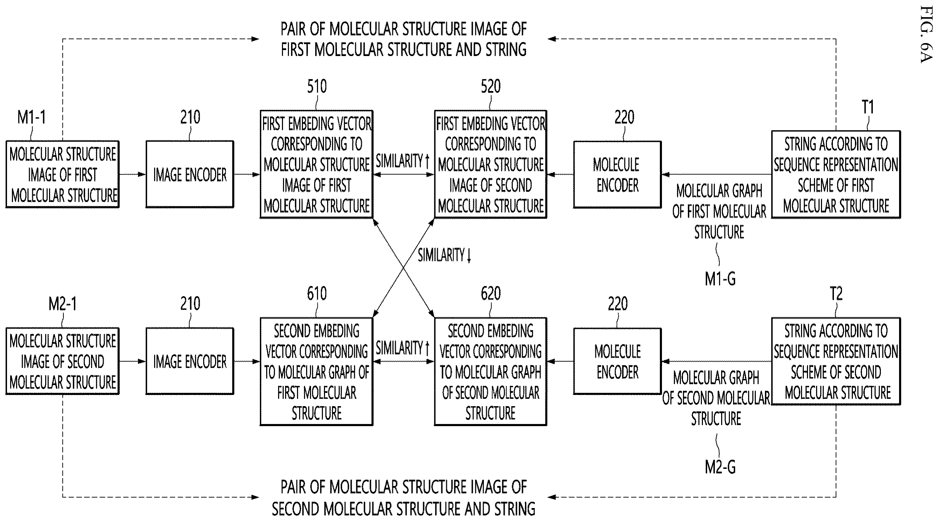

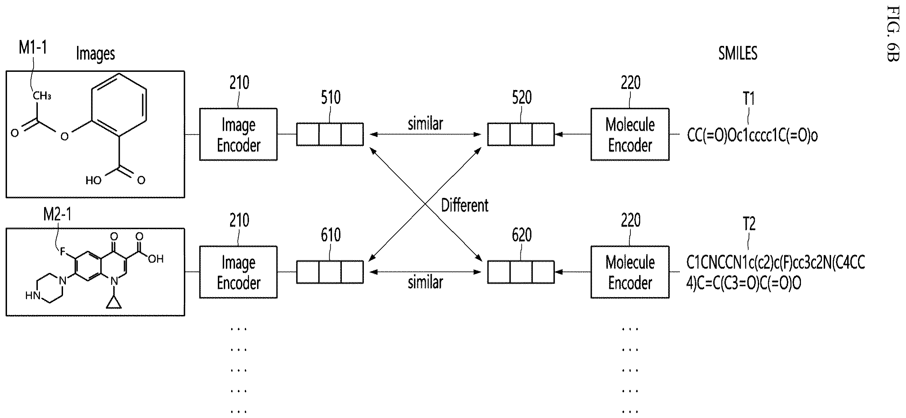

As illustrated in FIGS. 6A and 6B, the control unit 140 may input the molecular structure image M2-1 of the specified second molecular structure to the image encoder 210. For example, the control unit 140 may input at least one M2-1 of the plurality of molecular structure images having respectively different visual appearances of the second molecular structure to the image encoder 210.

Next, the control unit 140 may input the strings according to the sequence representation scheme of each of the plurality of molecular structures to the molecule encoder 220.

In this case, the strings of each of the plurality of molecular structures may be converted into the molecular graph and input to the molecule encoder 220. For example, the string T1 of the first molecular structure may be converted into the first molecular graph, and the string T2 of the second molecular structure may be converted into the second molecular graph, which may each be input to the molecule encoder 220.

Based on the strings according to the sequence representation scheme of each of the plurality of molecular structures, the control unit 140 may convert atoms into nodes and bonds between the atoms into edges to acquire the molecule graph of each of the plurality of molecular structures, and input the molecule graph of each of the plurality of molecular structures to the molecule encoder 220.

Specifically, as illustrated in FIGS. 5A and 5B, the control unit 140 may use the predefined tool (tool, or graph conversion unit 130) to convert atoms constituting the first molecular structure M1 among the plurality of molecular structures into nodes and convert bond relationships between the atoms constituting the first molecular structure into edges, thereby acquiring a first molecular graph M1-G including the nodes and edges corresponding to the first molecular structure M1.

In addition, as illustrated in FIGS. 6A and 6B, the control unit 140 may use the predefined tool to convert atoms constituting the second molecular structure M2 into nodes and convert bond relationships between the atoms constituting the second molecular structure M2 into edges, thereby acquiring a second molecular graph M2-G including the nodes and edges corresponding to the second molecular structure M2.

In addition, the control unit 140 may input the acquired first molecular graph M1-G and second molecular graph M2-G to the molecule encoder 220, respectively.

Furthermore, the control unit 140 may acquire, from the image encoder 210, embedding vectors corresponding to the molecular structure images of each of the plurality of molecular structures, and acquire, from the molecule encoder 220, embedding vectors corresponding to the strings according to the sequence representation scheme of each of the plurality of molecular structures.

The control unit 140 may acquire, from the image encoder 210, an embedding vector 510 (or a first embedding vector, an image embedding vector, etc.) corresponding to the molecular structure image M1-1 of the first molecular structure, and may acquire an embedding vector 610 (or a first embedding vector, an image embedding vector, etc.) corresponding to the molecular structure image M2-1 of the second molecular structure from the image encoder 210.

The process of acquiring the embedding vectors corresponding to each of the plurality of molecular structures described above may include a process of acquiring, from the image encoder 210, the embedding vectors corresponding to each of the plurality of molecular structure images having respectively different visual appearances of the first molecular structure and the embedding vectors corresponding to each of the plurality of molecular structure images having respectively different visual appearances of the second molecular structure.

In addition, the control unit 140 may acquire the embedding vectors corresponding to each of the first molecule graph M1-G and the second molecule graph M2-G from the molecule encoder 220.

The molecule encoder 220 may generate an embedding vector 520 (or a second embedding vector, a graph embedding vector, a molecule embedding vector, etc.,) corresponding to the first molecule graph M1-G using the nodes and edges corresponding to the first molecule graph M1-G. In addition, the molecule encoder 220 may generate an embedding vector 620 (or a second embedding vector, a graph embedding vector, a molecule embedding vector, etc. corresponding to the second molecule graph M2-G using the nodes and edges corresponding to the second molecule graph M2-G.

Through this, the control unit 140 may acquire the embedding vector 520 corresponding to the first molecular graph M1-G generated from the molecule encoder 220 and the embedding vector 620 corresponding to the second molecular graph M2-G, respectively.

Furthermore, the control unit 140 may store, in the storage unit (or memory, 120), the embedding vectors 510 and 610 corresponding to the molecular structure images M1-1 and M2-1 of each of the plurality of molecular structures, and the embedding vectors 520 and 620 corresponding to the strings T1 and T2 according to the sequence representation scheme of each of the plurality of molecular structures.

Meanwhile, the control unit 140 may perform the contrastive learning on the molecular structure recognition model 200 of the molecular structure recognition system 100 using the embedding vectors 510 and 610 corresponding to molecular structure images M1-1 and M2-1 of each of the plurality of molecular structures and the embedding vectors 520 and 620 corresponding to strings T1 and T2 according to the sequence representation scheme of each of the plurality of molecular structures, in which the embedding vector 510 and 610 and the embedding vectors 520 and 620 are stored in the memory.

The control unit 140 may define a similarity matrix for the molecular structure images of each of the plurality of molecular structure forming the pair input to the molecular structure recognition model 200 and string pair of each of the plurality of molecular structures that form a pair, in which the molecular structure images and string pair are input to the molecular structure recognition model 200 . For example, the control unit 140 may define the similarity matrix for the molecular structure image M1-1 of the first molecular structure and the string T1 (or a pair of the molecular structure image and the string of the first molecular structure, a molecular structure image-string pair of the first molecular structure, etc.) of the first molecular structure that form a pair, and the molecular structure image M2-1 of the second molecular structure and the string T2 (or a pair of the molecular structure images and the string of the second molecular structure, the molecular structure image-string pair of the second molecular structure, etc.) of the second molecular structure that form a pair, in which the molecular structure image M1-1 and the string T1 and the molecular structure image M2-1 and the string T2 are input to the molecular structure recognition model 200.

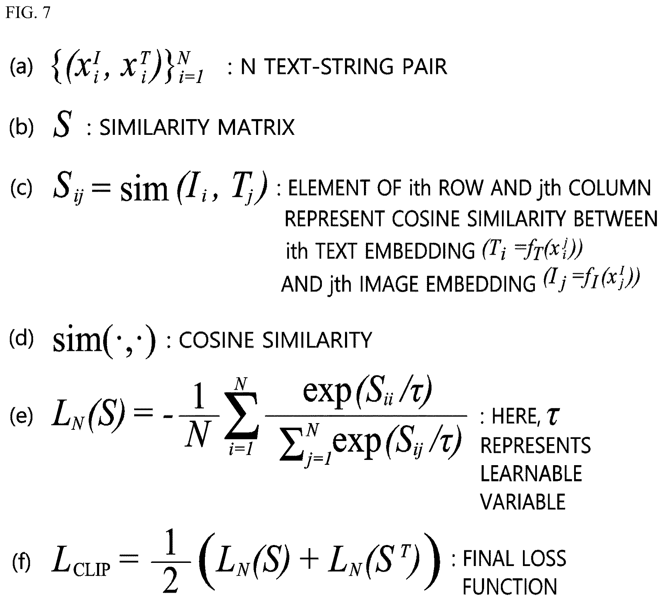

In the present disclosure, each pair of the molecular structure images and the strings of a plurality of molecular structures input to the molecular structure recognition model 200 may be represented as in (a) of FIG. 7, and a similarity matrix may be represented as in (b) of FIG. 7. In this case, the elements of the ith row and the jth column of the similarity matrix may be represented as in (c) of FIG. 7. This represents the similarity (e.g., cosine similarity) between ith text (or string) embedding and jth image embedding, and the cosine similarity may be represented as in (d) of FIG. 7.

The embedding I corresponding to the molecular structure image and the embedding corresponding to the string are projected into the same space (i.e., embedding space, vector space, etc.). In this case, the molecular structure recognition model 200 may be trained such that the positive pairs become closer to each other and the negative pairs become farther away from each other.

Here, the positive pair may include a pair of the molecular structure image and a string representing the same molecular structure, while the negative pair may include a pair of a molecular structure image and a string representing repectively different molecular structures.

More specifically, the positive pair may include an image representing the same molecular structure and a graphical representation (molecular graph) of the molecule. In other words, respectively different representations of the same molecule may exist, and the molecular structure recognition model 200 is trained to recognize the representations as identical.

For example, all molecular structure images generated having respectively different visual appearances for the same molecule described above may be considered (or defined) as positive pairs (or positive samples) corresponding to the same molecule. The molecular structure recognition model 200 is trained such that the representations of these samples are located close to each other, thereby understanding that various representations of the same molecule all have the same meaning. The goal of this process is to minimize the embedding distance between the molecular structure image and the molecular graph representing the same molecule by maximizing (or increasing) the similarity.

Conversely, the negative pair may refer to the molecular structure image and the molecular graph representing different molecular structures. That is, since repectively different molecular structures have respectively different meanings or characteristics, the molecular structure recognition model 200 may be trained to ensure that the embedding between respectively different molecular structures is located far apart.

For example, when the pair of the molecular structure image and the string of the first molecular structure is input to the molecular structure recognition model 200, and the molecular structure recognition model 200 performs the learning on the pair of the molecular structure image and the string of the first molecular structure, the pair of the molecular structure image and the string of the first molecular structure may be defined (or regarded) as the positive pair. On the other hand, the pair of the molecular structure image and the string of the second molecular structure may be defined as the negative pair. The molecular structure recognition model 200 may be trained such that the representations of the samples are located far apart, thereby clearly distinguishing the differences between the molecular structures. In this process, the goal is to maximize (i.e., increase) the embedding distance between the molecular structure image representing respectively different molecules and the molecular graph by minimizing (or decreasing) the similarity between the pair of molecular structure image and the string of the first molecular structure and the pair of molecular structure image and the string of the second molecular structure.

That is, the molecular structure recognition model 200 may be trained to maximize the similarity for the pair of the molecular structure image and the string of the same molecular structure and minimize the similarity for the pair of the molecular structure image and the string of respectively different molecular structures.

Meanwhile, given a similarity matrix S, a preset loss function for training (i.e., single encoder training) any one of the image encoder 210 or the molecule encoder 220 included in the molecular structure recognition model 200 of the present disclosure may be represented as illustrated in (e) of FIG. 7. This loss function in (e) of FIG. 7 may also be referred to as a "preset first loss function". Hereinafter, the description will be made on the assumption that the image encoder 210 is trained using the first loss function. However, the encoder trained using the first loss function is not necessarily limited to the image encoder 210. The molecule encoder 220 may also be trained using the first loss function.

The control unit 140 may perform the contrastive learning on at least one of the image encoder 210 and the molecule encoder 220 using the preset loss function.

Specifically, as illustrated in FIGS. 6A and 6B, the control unit 140 may perform training on at least one of the image encoder 210 and the molecule encoder 220 included in the molecular structure recognition model 200 using the preset loss function to maximize the similarity between the molecular structure image M1-1 of the first molecular structure forming the pair and the string T1 according to the sequence representation scheme of the first molecular structure.

Here, maximizing the similarity between the molecular structure image of the first molecular structure and the string according to the sequence representation scheme of the first molecular structure that form a pair may mean maximizing the similarity between the embedding vector 510 corresponding to the molecular structure image M1-1 of the first molecular structure and the embedding vector 520 corresponding to the first molecular graph M1-G such that the embedding vector 510 corresponding to the molecular structure image M1-1 of the first molecular structure and the embedding vector 520 corresponding to the first molecular graph M1-G are included in the first embedding space.

The control unit 140 may train the image encoder 210 in a direction in which the similarity between the embedding vector 510 corresponding to the molecular structure image M1-1 of the first molecular structure and the embedding vector 520 corresponding to the first molecular graph is maximized such that the embedding vector 510 corresponding to the molecular structure image M1-1 of the first molecular structure and the embedding vector 520 corresponding to the first molecular graph M1-G are included in the first embedding space.

That is, the control unit 140 may train the image encoder 210 in a direction in which the similarity between the embedding vectors corresponding to each of the plurality of molecular structure images having respectively different visual appearances of the first molecular structure and the embedding vectors corresponding to the first molecular graph M1-G for the first molecular structure is maximized such that the embedding vectors corresponding to each of the plurality of molecular structure images having respectively different visual appearances of the first molecular structure and the embedding vectors corresponding to the first molecular graph M1-G of the first molecular structure are located (or included) in the first embedding space.

Accordingly, the molecular structure image of the first molecular structure and the molecular graph of the first molecular structure are mapped to vector representations having the same meaning, and the embedding vectors corresponding to each of the plurality of molecular structure images having respectively different visual appearances of the first molecular structure M1 and the embedding vectors corresponding to the first molecular graph (or the string of the first molecular structure) may be clustered based on the structural similarity in the first embedding space corresponding to the first molecular structure M1. Alternatively, the molecular structure image (or the molecular structure image having respectively different visual appearances) of the first molecular structure and the first molecular graph trained through the molecular structure recognition model 200 may be grouped (or sorted) based on the similarity in the first embedding space.

In addition, as illustrated in FIGS. 6A and 6B, the control unit 140 may perform training on at least one of the image encoder 210 and the molecule encoder 220 included in the molecular structure recognition model 200 using the loss function so as to maximize the similarity between the molecular structure image M2-1 of the second molecular structure and the string T2 according to the sequence representation scheme of the second molecular structure that form a pair.

Here, maximizing the similarity between the molecular structure image M2-1 of the second molecular structure and the string T2 according to the sequence representation scheme of the second molecular structure that form a pair may mean maximizing the similarity between the embedding vector 610 corresponding to the molecular structure image M2-1 of the second molecular structure and the embedding vector 620 corresponding to the second molecular graph M2-G such that the embedding vector 610 corresponding to the molecular structure image M2-1 of the second molecular structure and the embedding vector 620 corresponding to the second molecular graph M2-G are included in the second embedding space.

The control unit 140 may train the image encoder 210 in the direction in which the similarity between the embedding vector 610 corresponding to the molecular structure image M2-1 of the second molecular structure and the embedding vector 620 corresponding to the second molecular graph M2-G is maximized such that the embedding vector 610 corresponding to the molecular structure image M2-1 of the second molecular structure and the embedding vector 620 corresponding to the second molecular graph M2-G are included in the second embedding space.

That is, the control unit 140 may train the image encoder 210 in a direction in which the similarity between the embedding vectors corresponding to each of the plurality of molecular structure images having respectively different visual appearances of the second molecular structure and the embedding vectors corresponding to the second molecular graph of the second molecular structure is maximized such that the embedding vectors corresponding to each of the plurality of molecular structure images having respectively different visual appearances of the second molecular structure and the embedding vectors corresponding to the second molecular graph M2-G of the second molecular structure are located (or included) in the second embedding space.

Accordingly, the molecular structure image of the second molecular structure and the molecular graph of the second molecular structure are mapped to vector representations having the same meaning, and the embedding vectors corresponding to each of the plurality of molecular structure images having respectively different visual appearances of the second molecular structure M2 and the embedding vectors corresponding to the second molecular graph (or the string of the second molecular structure) may be clustered based on the structural similarity in the second embedding space corresponding to the second molecular structure M2. Alternatively, the molecular structure image (or the molecular structure image having respectively different visual appearances) of the second molecular structure and the second molecular graph trained through the molecular structure recognition model 200 may be grouped (or sorted) based on the similarity in the second embedding space.

The similarity maximization process described above may be understood as training the molecular structure recognition model 200 to increase the similarity between the embedding vector corresponding to the molecular structure image of the positive pair and the embedding vector corresponding to the molecular graph.

Meanwhile, as illustrated in FIGS. 6A and 6B, the control unit 140 may perform training on the image encoder 210 included in the molecular structure recognition model 200 using the loss function so as to minimize the similarity between the molecular structure image M1-1 of the first molecular structure and the string T1 according to the sequence representation scheme of the first molecular structure that form a pair and the molecular structure image M2-1 of the second molecular structure and the string T2 according to the sequence representation scheme of the second molecular structure that form a pair.

Here, minimizing the similarity between the molecular structure image M1-1 of the first molecular structure and the string T1 according to the sequence representation scheme of the first molecular structure that form a pair and the molecular structure image M2-1 of the second molecular structure and the string T2 according to the sequence representation scheme of the second molecular structure that form a pair may mean minimizing the similarity between the embedding vector 610 corresponding to the molecular structure image M2-1 of the second molecular structure and the embedding vector 620 corresponding to the second molecular graph M2-G that are different from the similarity between the embedding vector 510 corresponding to the molecular structure image M1-1 of the first molecular structure and the embedding vector 520 corresponding to the first molecular graph M1-G .

Accordingly, the molecular structure image-string pair of the first molecular structure and the molecular structure image-string pair of the second molecular structure are distinguished (or discriminated, classified, separated, etc.) as vector representations having respectively different meanings, and the molecular structure recognition model (or image encoder, 200) may perform training on the vector representations.

The similarity minimization process described above may be understood as training the molecular structure recognition model 200 to decrease the similarity between the embedding vector corresponding to the molecular structure image of the negative pair and the embedding vector corresponding to the molecular graph.

Meanwhile, the preset loss function for bidirectional learning of the image encoder 210 and molecule encoder 220 included in the molecular structure recognition model of the present disclosure may be represented as illustrated in (f) of FIG. 7. This loss function in (f) of FIG. 7 may also be referred to as a "preset second loss function".

Here, the bidirectional learning may be understood as performing symmetrical learning from the molecular structure image to the molecular graph, or from the molecular graph to the molecular structure image. While the loss function in (e) of FIG. 7 described above is a loss function for training a single encoder, the loss function in (f) of FIG. 7 may be a loss function that considers the bidirectional learning of the encoder.

That is, the control unit 140 may train the image encoder 210 and the molecule encoder 220 included in the molecular structure recognition model 200 using the loss function in (f) of FIG. 7 to train which molecular graph (molecular structure direction from the molecular structure image) the input molecular structure is connected to, or which molecular structure image (molecular structure direction from the molecular structure) the input molecular structure is connected to.

The molecular structure recognition model 200 may be trained to recognize the molecular structures corresponding to the molecular structure images of each of the plurality of molecular structures, the molecular structures corresponding to the strings according to the sequence representation scheme of each of the plurality of molecular structures, or the molecular structures corresponding to the molecular graph of each of the plurality of molecular structures, using the loss function in (f) of FIG. 7.

The molecular structure recognition model 200 (or image encoder) trained through the learning process described above may extract meaningful image features (or embedding vectors) from the molecular structure image. That is, the features related to the molecular structure image (or molecular structural formula, molecular structure) may be extracted.

In this way, as illustrated in FIG. 4, the learning process of the molecular structure recognition model according to the present disclosure may include a process (S410) of inputting the molecular structure images of each of the plurality of molecular structures to the image encoder, a process (S420) of inputting the strings according to the sequence representation scheme of each of the plurality of molecular structures to the molecule encoder, a process (S430) of acquiring the embedding vectors corresponding to the molecular structure images of each of the plurality of molecular structures from the image encoder, a process (S440) of acquiring the embedding vectors corresponding to the strings according to the sequence representation scheme of each of the plurality of molecular structures from the molecule encoder, and a process (S450) of storing, in the memory, the embedding vectors corresponding to the molecular structure images of each of the plurality of molecular structures and the embedding vectors corresponding to the strings according to the sequence representation scheme of each of the plurality of molecular structures. Furthermore, the learning process may include a process (S460) of performing the contrastive learning on the molecular structure recognition model 200 of the molecular structure recognition system 100 using the embedding vectors corresponding to the molecular structure images of each of the plurality of molecular structures and the embedding vectors corresponding to the strings according to the sequence representation scheme of each of the plurality of molecular structures that are stored in the memory.

Meanwhile, the molecular structure recognition model 200 trained according to the learning method described above may be configured at the inference stage, as illustrated in FIG. 10, without the molecule encoder 220. As illustrated in FIG. 8, in the inference stage, the control method of a molecular structure recognition system according to the present disclosure may include a process (S810) of receiving a user query related to the specific molecular structure among the plurality of molecular structures from the user terminal, a process (S820) of processing the user query as input to the molecular structure recognition model trained based on the contrastive learning between the embedding vectors corresponding to the molecular structure images of each of the plurality of molecular structures and the embedding vectors corresponding to the strings according to the sequence representation scheme of each of the plurality of molecular structures, a process (S830) of extracting information on the specific molecular structure corresponding to the user query from the molecular structure recognition model, a process (S840) of processing the extracted information on the specific molecular structure as input to the large language model (LLM), a process (S850) of acquiring a response to the user query from the large language model, and a process (S860) of providing the response to the user query to the user terminal.

Here, the user query may include at least one of the molecular structure image of the molecular structure, the string according to the sequence representation scheme of the molecular structure, the graph of the molecular structure, the molecular structural formula, and a description related to the molecular structure. For example, as illustrated in FIG. 9, the control unit 140 may receive the user query 901 (e.g., "Explain about the corresponding molecular structure") including a molecular structure image 901a through a service page (or USER TERMINAL).

In an embodiment, as illustrated in FIG. 10, the control unit 140 may input the molecular structure image 901a included in the user query 901 to the image encoder 210 of the molecular structure recognition model 200 trained based on the contrastive learning between the embedding vectors corresponding to the molecular structure images of each of the plurality of molecular structures and the embedding vectors corresponding to the strings according to the sequence representation scheme of each of the plurality of molecular structures. When the specific molecular structure image 901a is input, the image encoder 210 may extract the information on the specific molecular structure corresponding to the molecular structure image 901a from the storage unit 120 or DB, and process the extracted information on the specific molecular structure as input to a large language model 1000. Furthermore, the control unit 140 may acquire the response 910 to the user query 901 (e.g., "This molecular structural formula chemically appears to be one of derivatives of indole-3-carboxylic acid. Its core structure has an indole ring structure, which is a fused structure of a benzene ring and a pyrrole ring...") from the large language model 1000, and provide a response to a user query 901 generated from the large language model 1000 to the USER TERMINAL.

In another embodiment, as illustrated in FIG. 1, the molecular structure recognition model 200 may receive a molecular structure image 1 of a molecular structure as input to a user query, and recognize a molecular structure corresponding to the molecular structure image 1. Then, the control unit 140 may search for a document (e.g., a patent document, a paper, etc., D) including a molecular structure 2 corresponding to the molecular structure image 1 based on the recognized result, and provide the searched document D to the USER TERMINAL.

In another embodiment, the molecular structure recognition model 200 may receive the string according to the sequence representation scheme of the molecular structure as input to the user query and may recognize the molecular structure corresponding to the string of the molecular structure. Then, the control unit 140 may search the document including the molecular structure corresponding to the molecular structure string based on the recognition result and provide the searched document to the USER TERMINAL.

As described above, according to the molecular structure recognition system, the control method thereof, and the learning method of a molecular structure recognition system according to the present disclosure, it is possible to perform the contrastive learning on the molecular structure recognition model using the embedding vectors corresponding to the molecular structure image of each of the plurality of molecular structures and the embedding vectors corresponding to strings according to the sequence representation scheme of each of the plurality of molecular structures. The molecular structure recognition model trained through this may accurately recognize molecules even in various styles of molecular structure images and changes in orientation, and accurately distinguish subtle molecular structure differences even in molecular structure images including small differences such as atomic changes, bond changes, chirality changes, and the like. In addition, the molecular structure recognition model may precisely analyze the similarities and differences between molecules by clearly identifying gradual differences (or degrees of modification) from small structural changes to large changes in molecules.

In addition, the molecular structure recognition system, the control method thereof method, and the learning method of a molecular structure recognition system according to the present disclosure may perform training to maximize the similarity between the pairs of the molecular structure images and the strings of the same molecular structure, and minimize the similarity between the pairs of the molecular structure images and the strings of respectively different molecular structures. The encoder of the molecular structure recognition model trained through this method may extract meaningful features including a deep understanding of the molecular structures from the molecular structural formula images without the need for direct image-to-molecule conversion. In addition, the encoder of the molecular structure recognition model can more accurately identify the details of the complex molecules and minimize the information loss during the translation process. In other words, according to the present disclosure, by enabling the direct understanding and interpretation of the molecular images, it is possible to significantly expand its applicability in the fields of chemistry research.

Furthermore, according to the molecular structure recognition system, the control method thereof, and the learning method of a molecular structure recognition system according to the present disclosure, it is possible to construct the training dataset by generating the molecular structure images having respectively different visual appearances while maintaining the characteristics of each of the plurality of molecular structures. In other words, according to the present disclosure, it is possible to provide the environment in which the model may perform training from various styles of molecular structure images while maintaining structural consistency of molecules. Through this, the molecular structure recognition model may utilize various styles of molecular structure images as training data, thereby maintaining consistent molecular recognition performance across diverse styles of molecular structure images without being biased toward a specific style of molecular structure image. In other words, the molecular structure recognition model may be widely utilized in patent search, new drug development, and chemical research, etc., by maintaining robust molecular recognition capabilities through training on various styles of molecular structure images.

In this way, the molecular structure recognition model according to the present disclosure may more clearly maintain structural similarities between molecules even in various styles of images. In particular, the encoder of the molecular structure recognition model may be robust to style changes and effectively distinguish the molecular structures. In addition, it is possible to achieve the strong generalization performance even on untrained new molecular structures and effectively recognize and search molecules through the image embedding. In other words, the molecular structure recognition model according to the present disclosure may easily understand the structural characteristics of molecules from the molecular structure image itself, just as humans easily understand the molecular structure images, thereby effectively extracting molecular-related features even for complex molecular structures.

Meanwhile, as described above, the present disclosure may be implemented as a program that is executed by one or more processes on a computer and stored on a computer-readable medium (or recording medium).

Furthermore, as described above, the present disclosure may be implemented as computer-readable codes or instructions on a medium recording the program. In other words, the present disclosure may be provided in the form of the program.

Meanwhile, the computer readable medium may include all kinds of recording devices in which computer system-readable data is stored. An example of the computer readable medium may include a hard disk drive (HDD), a solid state disk (SSD), a silicon disk drive (SDD), a read only memory (ROM), a random access memory (RAM), a compact disk read only memory (CD-ROM), a magnetic tape, a floppy disk, an optical data storage, and the like.

Furthermore, the computer-readable medium may be the server or cloud storage that includes storage and may be accessed by the electronic device via communication. In this case, the computer may download the program according to the present disclosure from the server or cloud storage via wired or wireless communication.

Furthermore, in the present disclosure, the computer described above is an electronic device equipped with a processor, i.e., a central processing unit (CPU), and there are no particular limitations on its type.

Meanwhile, the above-described detailed description is to be interpreted as being illustrative rather than being restrictive in all aspects. The scope of the present disclosure is to be determined by reasonable interpretation of the claims, and all modifications within an equivalent range of the present disclosure fall in the scope of the present disclosure.

## Claims

1. A learning method of a molecular structure recognition system, performed cooperatively by a memory and at least one processor, the learning method comprising:
inputting molecular structure images of each of a plurality of molecular structures to an image encoder;
inputting strings according to a sequence representation scheme of each of the plurality of molecular structures to a molecule encoder;
acquiring, from the image encoder, embedding vectors corresponding to the molecular structure images of each of the plurality of molecular structures;
acquiring, from the molecule encoder, the embedding vectors corresponding to the strings according to the sequence representation scheme of each of the plurality of molecular structures;
storing, in the memory, the embedding vectors corresponding to the molecular structure images of each of the plurality of molecular structures and the embedding vectors corresponding to the strings according to the sequence representation scheme of each of the plurality of molecular structures; and
performing contrastive learning on a molecular structure recognition model of the molecular structure recognition system using the embedding vectors corresponding to the molecular structure images of each of the plurality of molecular structures and the embedding vectors corresponding to the strings according to the sequence representation scheme of each of the plurality of molecular structures that are stored in the memory.

2. The learning method of claim 1, further comprising:
constructing a training dataset including the molecular structure images of each of the plurality of molecular structures and the strings according to the sequence representation scheme of each of the plurality of molecular structures,
wherein the training dataset is constructed such that the molecular structure images of each of the plurality of molecular structures and the strings according to the sequence representation scheme of each of the plurality of molecular structures form a pair.

3. The learning method of claim 2, wherein at least one molecular structure image having respectively different visual appearances is paired with each string according to the sequence representation scheme of each of the plurality of molecular structures.

4. The learning method of claim 3, wherein the molecular structure images of each of the plurality of molecular structures include the molecular structure images having the respectively different visual appearances generated using a predefined molecular structure image generation scheme, and
in the constructing of the training dataset, the molecular structure images having the respectively different visual appearances while maintaining characteristics of each of the plurality of molecular structures are generated.

5. The learning method of claim 2, further comprising:
specifying, from the training dataset, the molecular structure images of each of the plurality of molecular structures and the strings according to the sequence representation scheme of each of the plurality of molecular structures that form a pair,
wherein the plurality of molecular structures include at least one of a first molecular structure and a second molecular structure.

6. The learning method of claim 5, wherein the specifying includes:
specifying a molecular structure image of the first molecular structure and a string according to a sequence representation scheme of the first molecular structure that form a pair; and
specifying a molecular structure image of the second molecular structure and a string according to a sequence representation scheme of the second molecular structure that form a pair.

7. The learning method of claim 1, wherein the inputting of the strings to the molecule encoder includes:
converting atoms into nodes and converting bonds between the atoms into edges based on the strings according to the sequence representation scheme of each of the plurality of molecular structures to acquire a molecular graph of each of the plurality of molecular structures; and
inputting the molecular graph of each of the plurality of molecular structures to the molecule encoder.

8. The learning method of claim 7, wherein the acquiring of the molecular graph includes:
converting atoms constituting a first molecular structure into nodes and converting bond relationships between the atoms constituting the first molecular structure into edges using a predefined tool to acquire a first molecular graph including the nodes and edges corresponding to the first molecular structure; and
converting atoms constituting a second molecular structure into nodes and converting bond relationships between the atoms constituting the second molecular structure into edges using the tool to acquire a second molecular graph including the nodes and edges corresponding to the second molecular structure, and
in the inputting to the molecule encoder, the acquired first molecule graph and second molecule graph are input to the molecule encoder.

9. The learning method of claim 8, wherein, in the molecule encoder, an embedding vector corresponding to the first molecule graph is generated using the nodes and edges corresponding to the first molecule graph,
in the molecule encoder, an embedding vector corresponding to the second molecule graph is generated using the nodes and edges corresponding to the second molecule graph, and
the embedding vector corresponding to the first molecule graph and the embedding vector corresponding to the second molecule graph generated from the molecule encoder are acquired.

10. The learning method of claim 1, wherein, in the inputting of the molecular structure images to the image encoder,
a molecular structure image of a first molecular structure and a molecular structure image of a second molecular structure are input to the image encoder, and
an embedding vector corresponding to the molecular structure image of the first molecular structure and an embedding vector corresponding to the molecular structure image of the second molecular structure are acquired from the image encoder.

11. The learning method of claim 1, wherein, in the performing of the contrastive learning,
the molecular structure recognition model is trained using a preset loss function so as to maximize a similarity between a molecular structure image of a first molecular structure and a string according to a sequence representation scheme of the first molecular structure that form a pair, and
the molecular structure recognition model is trained using the loss function so as to maximize a similarity between a molecular structure image of a second molecular structure and the string according to the sequence representation scheme of the second molecular structure that form a pair.

12. The learning method of claim 11, wherein a similarity between an embedding vector corresponding to the molecular structure image of the first molecular structure and an embedding vector corresponding to a first molecular graph is maximized so that the embedding vector corresponding to the molecular structure image of the first molecular structure and the embedding vector corresponding to the first molecular graph are included in a first embedding space, and
a similarity between an embedding vector corresponding to the molecular structure image of the second molecular structure and an embedding vector corresponding to a second molecular graph is maximized so that the embedding vector corresponding to the molecular structure image of the second molecular structure and the embedding vector corresponding to the second molecular graph are included in a second embedding space.

13. The learning method of claim 1, wherein, in the performing of the contrastive learning, the molecular structure recognition model is trained using a loss function so as to minimize a similarity between a molecular structure image of a first molecular structure and a string according to a sequence representation scheme of the first molecular structure that form a pair, and a molecular structure image of a second molecular structure and a string according to a sequence representation scheme of a second molecular structure that form a pair.

14. The learning method of claim 13, wherein the similarity between the embedding vector corresponding to the molecular structure image of the second molecular structure and the embedding vector corresponding to the second molecular graph, which is different from the similarity between the embedding vector corresponding to the molecular structure image of the first molecular structure and the embedding vector corresponding to the first molecular graph, is minimized.

15. The learning method of claim 14, wherein the molecular structure recognition model is trained to recognize the molecular structures corresponding to the molecular structure images of each of the plurality of molecular structures, recognize the molecular structures corresponding to the strings according to the sequence representation scheme of each of the plurality of molecular structures, or recognize molecular structures corresponding to a molecular graph of each of the plurality of molecular structures.

16. A molecular structure recognition system, comprising:
a memory and at least one processor,
wherein the memory and the processor cooperate to:
input molecular structure images of each of a plurality of molecular structures to an image encoder;
input strings according to a sequence representation scheme of each of the plurality of molecular structures to a molecule encoder;
acquire, from the image encoder, embedding vectors corresponding to the molecular structure images of each of the plurality of molecular structures;
acquire, from the molecule encoder, the embedding vectors corresponding to the strings according to the sequence representation scheme of each of the plurality of molecular structures; and
perform contrastive learning on a molecular structure recognition model of the molecular structure recognition system using the embedding vectors corresponding to the molecular structure images of each of the plurality of molecular structures and the embedding vectors corresponding to the strings according to the sequence representation scheme of each of the plurality of molecular structures.

17. A program stored on a computer-readable recording medium, executed by one or more processes in an electronic device, wherein the program includes instructions to perform:
inputting molecular structure images of each of a plurality of molecular structures to an image encoder;
inputting strings according to a sequence representation scheme of each of the plurality of molecular structures to a molecule encoder;
acquiring, from the image encoder, embedding vectors corresponding to the molecular structure images of each of the plurality of molecular structures;
acquiring, from the molecule encoder, the embedding vectors corresponding to the strings according to the sequence representation scheme of each of the plurality of molecular structures; and
performing contrastive learning on a molecular structure recognition model of the molecular structure recognition system using the embedding vectors corresponding to the molecular structure images of each of the plurality of molecular structures and the embedding vectors corresponding to the strings according to the sequence representation scheme of each of the plurality of molecular structures.

18. A control method of a molecular structure recognition system, comprising:
receiving, from a user terminal, a user query related to a specific molecular structure among a plurality of molecular structures;
processing the user query as input to a molecular structure recognition model trained based on contrastive learning between embedding vectors corresponding to molecular structure images of each of the plurality of molecular structures and embedding vectors corresponding to strings according to a sequence representation scheme of each of the plurality of molecular structures;
extracting information on the specific molecular structure corresponding to the user query from the molecular structure recognition model;
processing the extracted information on the specific molecular structure as input to a large language model (LLM);
acquiring a response to the user query from the large language model; and
providing the response to the user query to the user terminal.

19. A molecular structure recognition system, comprising:
a control unit configured to receive, from a user terminal, a user query related to a specific molecular structure among a plurality of molecular structures, and
process the user query as input to a molecular structure recognition model trained based on contrastive learning between embedding vectors corresponding to molecular structure images of each of the plurality of molecular structures and embedding vectors corresponding to strings according to a sequence representation scheme of each of the plurality of molecular structures,
wherein the control unit extracts information on the specific molecular structure corresponding to the user query using the molecular structure recognition model,
processes the extracted information on the specific molecular structure as input to a large language model (LLM),
acquires a response to the user query from the large language model; and
provides the response to the user query to the user terminal.

20. A program stored on a computer-readable recording medium, executed by one or more processors in an electronic device, wherein the program includes instructions to perform:
receiving, from a user terminal, a user query related to a specific molecular structure among a plurality of molecular structures;
processing the user query as input to a molecular structure recognition model trained based on contrastive learning between embedding vectors corresponding to molecular structure images of each of the plurality of molecular structures and embedding vectors corresponding to strings according to a sequence representation scheme of each of the plurality of molecular structures;
extracting information on the specific molecular structure corresponding to the user query from the molecular structure recognition model;
processing the extracted information on the specific molecular structure as input to a large language model (LLM);
acquiring a response to the user query from the large language model; and
providing the response to the user query to the user terminal.
